# EUROPÄISCHE PATENTSCHRIFT

(11) **EP 1 201 193 B1**
(45) Veröffentlichungstag und Bekanntmachung des Hinweises auf die Patenterteilung: **03.12.2008**
(21) Anmeldenummer: 01810913.2
(22) Anmeldetag: 20.09.2001
(51) Int. Cl.: A61B 17/28

(54) **Chirurgisches Instrument**
Surgical instrument
Instrument chirurgical

(30) Priorität: 19.10.2000 CH 20492000; 16.05.2001 CH 9082001
(43) Veröffentlichungstag der Anmeldung: 02.05.2002
(73) Patentinhaber: Alcon Grieshaber AG, 8203 Schaffhausen (CH)
(72) Erfinder: Etter, Heinz, 8408 Winterthur (CH); Attinger, Jürg, 8260 Stein am Rhein (CH); Maag, Werner, 8750 Glarus (CH)
(74) Vertreter: Spierenburg, Pieter

(56) Entgegenhaltungen:
- DE-U- 9 016 261
- US-A- 5 370 658
- US-A- 5 868 761
- US-A- 5 893 873
- US-A- 5 893 877

## Beschreibung

Die Erfindung bezieht sich auf ein chirurgisches Instrument, insbesondere zur Durchführung ophthalmologischer Eingriffe, bestehend aus einem Gehäuse mit zwei länglichen Gehäuseteilen sowie einem in Längsrichtung orientiert dazwischen angeordneten Tragarm, welcher an dem einen Ende mit den beiden Gehäuseteilen wirkverbunden und an dem anderen gegenüberliegenden Ende zur Befestigung einer Funktionseinheit ausgebildet ist, wobei die Funktionseinheit einen Stössel sowie damit zusammenwirkende Klemm- oder Schneidelemente umfasst, die beim Zusammendrücken der beiden Gehäuseteile betätigbar sind, wobei eine an dem Tragarm angeordnete und mit den beiden Gehäuseteilen zusammenwirkende Übertragungsvorrichtung, mittels welcher die beim Zusammendrücken der beiden Gehäuseteile etwa quer zu dem Tragarm orientierte Bewegung in eine axial in Richtung der Funktionseinheit orientierte Linearbewegung übersetzbar ist und infolge davon ein mit dem einen Ende an der Übertragungsvorrichtung angeordnetes und mit dem anderen Ende mit dem Stössel für die Betätigung der Klemm- oder Schneidelemente zusammenwirkendes Stellglied in axialer Richtung verschiebbar ist.

Aus der US-A 5,370,658 ist ein chirurgisches Instrument bekannt, welches ein als Handgriff ausgebildetes Gehäuse mit zwei länglichen relativ zueinander spreizbaren Gehäuseteilen, ein dazwischen angeordnetes und mit einem Endstück versehenes Tragrohr sowie ein daran angeordnetes Kopfstück zur aufschraubbaren Befestigung einer Funktionseinheit umfasst, wobei die Funktionseinheit eine röhrchenförmigen Sonde sowie eine in axialer Richtung darin angeordnete Stange aufweist, welche am distalen Ende beispielsweise mit einem als Schere ausgebildeten Werkzeug versehen ist. Für die Betätigung des Werkzeuges sind an den Innenseiten der Gehäuseteile zwei Hebel gelagert und derart mit einem Stellglied wirkverbunden, dass beim Zusammendrücken der beiden Gehäuseteile die Hebel das Stellglied in axialer Richtung verschieben und dabei das Werkzeug betätigen. Bei dieses chirurgischen Instrument ist beim Zusammendrücken der beiden Gehäuseteile eine seitliche Führung der relativ langen und lediglich an dem einen Ende zwischen den Wänden des gehäuseseitigen Lagerblocks geführte Hebel nicht vorgesehen, so dass die in axialer Richtung orientierte Linearbewegung zur Betätigung des Werkzeuges nicht präzise übertragbar ist.

Aus der US-A 5,893,877 ist ein ähnliches chirurgisches Instrument derselben Anmelderin bekannt, welches ähnlich wie vorhin ausgebildet ist, wobei nunmehr zwei flache spreizbare Gehäuseteile auf zwei lange Hebel einwirken. Die Hebelarme dieser Ausführung sind zwischen den Wänden der im Tragarm vorgesehenen Schlitzen geführt. Jedoch ist auch hier ebenfalls eine präsize Übertragung der Linearbewegung zur Betätigung des Werkzeuges nicht gewährleistet.

Aus der US-A 5,893,873 ist weiterhin ein chirurgisches Instrument bekannt, welches ein als Handgriff ausgebildetes Gehäuse mit zwei länglichen Gehäuseteilen, einen dazwischen angeordneten Tragarm für eine daran angeordnete Funktionseinheit sowie zwei in axialer Richtung orientierte und mit an den Innenseiten der Gehäuseteile angeordneten Hebeln zusammenwirkende Stellglieder umfasst, mittels welcher beim Zusammendrücken der beiden Gehäuseteile eine röhrchenförmige Sonde in axialer Richtung relativ zu einer Stange verschiebbar und infolge davon ein am distalen Ende derselben angeordnetes Klemmelement betätigbar ist.

Aus der Druckschrift DE 90 16 261 U ist ein mit zwei länglichen Federarmen versehenes Operationsinstrument bekannt, welches ein zwischen den beiden Federarmen angeordnetes Mittelteil, zwei daran gelagerte und damit wirkverbundene Hebel, ein an dem Mittelteil angeordnetes Kopfstück sowie eine darin gelagerte Sonde umfasst, mittels welcher ein am distalen Ende einer Stange angeordnetes Schneid- oder Klemmelement betätigbar ist.

Der Erfindung liegt somit die Aufgabe zugrunde, ein chirurgisches Instrument der eingangs genannten Art dahingehend zu verbessern, dass die beim Zusammendrücken der beiden Gehäuseteile bewirkte.

Bewegung exakt geführt und somit das für den chirurgischen Eingriff am distalen Ende der Sonde angeordnete Werkzeug exakt betätigbar ist.

Die vorstehende Aufgabe wird erfindungsgemäss durch die im kennzeichnenden Teil des Anspruchs 1 angegebenen Merkmale gelöst. Weitere Merkmale und zweckmässige weitere Ausgestaltungen sind in den Unteransprüchen angegeben.

Weiterhin wird bevorzugt, dass die beiden länglichen Gehäuseteile am proximalen Ende des Instruments derart mit dem Tragarm wirkverbunden sind, dass ein seitliches Ausschwenken der Gehäuseteile verhindert wird.

Weitere Merkmale und Ausführungsbeispiele der Erfindung ergeben sich aus der nachstehenden Beschreibung in Verbindung mit der Zeichnung und den einzelnen Patentansprüchen. Es zeigt:
- **Fig.1A**: ein räumlich sowie in grösserem Massstab dargestelltes erstes Ausführungsbeispiel eines chirurgischen Instruments mit einem als Handgriff ausgebildeten Gehäuse mit einer daran angeordneten Funktionseinheit;
- **Fig.1B**: das in schematischer Ansicht dargestellte chirurgische Instrument gemäss Fig.1 mit dem Gehäuse und der in demontiertem Zustand dargestellten Funktionseinheit;
- **Fig.1C**: das in Ansicht dargestellte chirurgische Instrument gemäss Fig.1B mit einer zwischen zwei Gehäuseteilen des Gehäuses angeordneten Übertragungsvorrichtung sowie die damit wirkverbundene Funktionseinheit;
- **Fig.1D**: ein schematisch dargestelltes Teilstück der Funktionseinheit mit einem in einer geöffneten Stellung dargestellten Klemmelement für das chirurgische Instrument;
- **Fig.1E**: das mit den beiden Klemmarmen versehene Klemmelement gemäss Fig.1D in einer geschlossenen Stellung;
- **Fig.1F**: ein schematisch und als Variante dargestelltes Teilstück der Funktionseinheit mit einem als Schere azsgebildeten Schneidelement für das chirurgische Instrument;
- **Fig.1G**: die beiden in grösserem Massstab und in schematischer Ansicht dargestellten Schneidblätter des Schneidelements gemäss Fig.1F;
- **Fig.2A**: ein in grösserem Massstab sowie im Schnitt dargestelltes erstes Ausführungsbeispiel des mit zwei Gehäuseteilen und einem Tragarm sowie einer Übertragungsvorrichtung versehenen Gehäuses für das chirurgische Instrument gemäss Fig. 1A;
- **Fig.2B**: das Gehäuse gemäss Fig.2A mit den beiden am vorderen Ende relativ zu dem Tragarm gespreizt dargestellten Gehäuseteilen sowie die dazwischen angeordnete Obertragungsvorrichtung;
- **Fig.2C**: das gemäss der in Fig.2A eingezeichneten Linie II-II im Schnitt dargestellte Gehäuse mit den beiden Gehäuseteilen und die dazwischen angeordnete übertragungsvorrichtung;
- **Fig.3A**: das eine im Schnitt dargestellte Gehäuseteil des Gehäuses gemäss Fig.2A;
- **Fig.3B**: das im Profilquerschnitt dargestellte Gehäuseteil gemäss der Linie III-III in Fig.3A;
- **Fig.4A**: ein in Ansicht dargestelltes Stellglied für die Obertragungsvorrichtung gemäss Fig.2A bis 2C;
- **Fig.4B**: das in Seitenansicht dargestellte Stellglied gemäss Fig.4A;
- **Fig.5A**: den in Draufsicht dargestellten Tragarm für die Funktionseinheit;
- **Fig.5B**: den im Schnitt und teilweise in Ansicht dargestellten Tragarm gemäss Fig.5A;
- **Fig.5C**: den gemäss der in Fig.5B eingezeichneten Linie V-V im Profilquerschnitt dargestellten Tragarm;
- **Fig.6A**: eine in schematischer Ansicht dargestellte Variante des chirurgischen Instruments mit dem als Handgriff ausgebildeten Gehäuse und die am vorderen Ende daran angeordnete Funktionseinheit;
- **Fig.7A**: ein in grösserem Massstab sowie im Schnitt dargestelltes zweites Ausführungsbeispiel des mit zwei Gehäuseteilen und einem Tragarm sowie einer zweiten Übertragungsvorrichtung versehenen Gehäuses für das chirurgische Instrument gemäss Fig.6A;
- **Fig.7B**: das Gehäuse gemäss Fig.7A mit den beiden am vorderen Ende relativ zu dem Tragarm aufgeschwenkt dargestellten Gehäuseteilen und die dazwischen angeordnete zweiten Übertragungsvorrichtung;
- **Fig.7C**: das gemäss der Linie VII-VII in Fig.7A im Schnitt dargestellte Gehäuse mit den beiden Gehäuseteilen, dem Tragarm und die dazwischen angeordnete zweite Übertragungsvorrichtung;
- **Fig.7D**: das im Profilquerschnitt dargestellte erste Gehäuseteil für das Gehäuse gemäss Fig. 7A;
- **Fig.7E**: das erste Gehäuseteil gemäss Fig.7C mit einer Führungsbahn für die zweite Übertragungsvorrichtung;
- **Fig.8A**: ein im Schnitt dargestelltes Teilstück des zweiten Gehäuseteils für das Gehäuse gemäss Fig.7A;
- **Fig.8B**: das gemäss der Linie VIII-VIII in Fig.8A im Profilquerschnitt dargestellte zweite Gehäuseteil mit einer daran angeordneten Lagerung für die zweite Übertragungsvorrichtung;
- **Fig.8C**: das zweite Gehäuseteil gemäss Fig.8B mit einer Führungsbahn für die zweite Übertragungsvorrichtung;
- **Fig.9A**: ein Teilstück des im Schnitt dargestellten Tragarms mit am vorderen Ende angeordnetem Kopfstück für die Funktionseinheit;
- **Fig.9B**: das in Draufsicht dargestellte Teilstück des in Fig.9A dargestellten Tragarms;
- **Fig.10A**: die in Ansicht sowie in grösserem Massstab dargestellte zweite Übertragungsvorrichtung für das chirurgische Instrument gemäss Fig.6A;
- **Fig.10B**: die in Draufsicht dargestellte Übertragungsvorrichtung gemäss Fig. 10A;
- **Fig.11A**: die im Schnitt sowie in grösserem Massstab dargestellte erste Funktionseinheit gemäss Fig.1D mit dem in der Offenstellung dargestellten Klemmelement;
- **Fig.11B**: die Funktionseinheit gemäss Fig.11A mit den beiden in geschlossener Stellung dargestellten Klemmarmen; und
- **Fig.11C**: die im Schnitt sowie in grösserem Massstab dargestellte zweite Funktionseinheit gemäss Fig.1F mit dem Schneidelement.

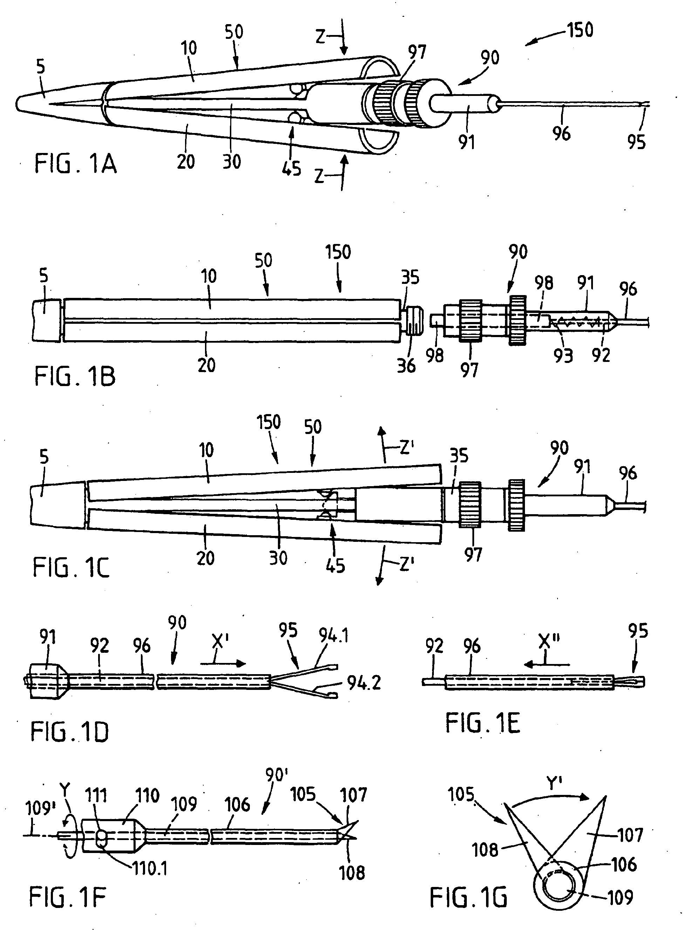

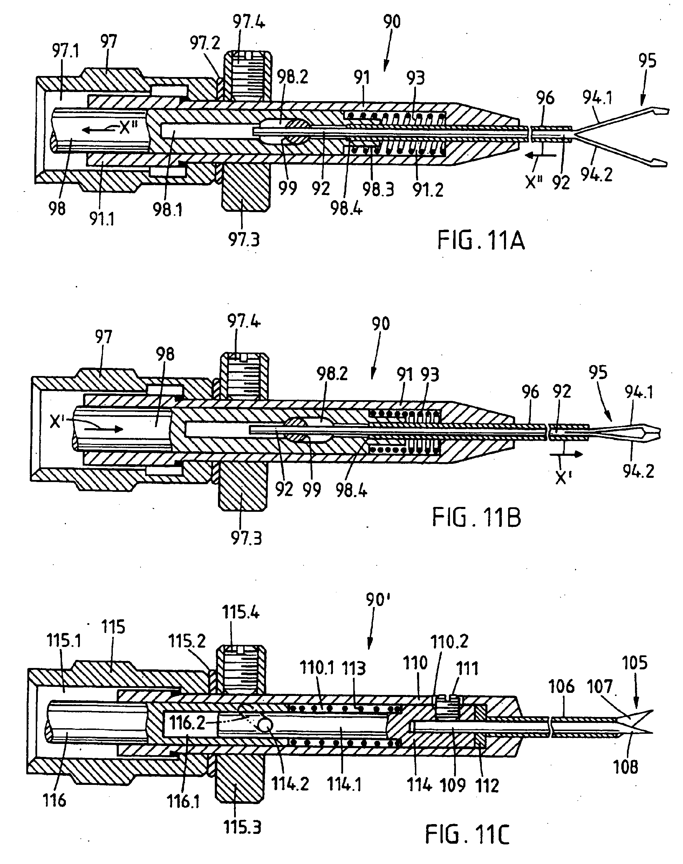

Fig.1A zeigt als erstes Ausführungsbeispiel ein räumlich dargestelltes und in der Gesamtheit mit 150 bezeichnetes chirurgisches Instrument. Das beispielsweise zur Durchführung ophthalmologischer Eingriffe ausgebildete Instrument 150 umfasst ein als Handgriff ausgebildetes und mit zwei Gehäuseteilen 10 und 20 sowie einem dazwischen angeordneten Tragarm 30 versehenes Gehäuse 50. An dem hinteren Ende des Gehäuses 50 ist eine beispielsweise aufsteckbar ausgebildete Verschlusskappe 5 angeordnet. An dem vorderen Ende des Tragarms 30 ist eine mit einem schematisch dargestellten Klemmelement 95 versehene Funktionseinheit 90 angeordnet. Die erste Funktionseinheit 90 ist mit dem einen Ende an einem Kopfstück 35 des Tragarms 30 angeordnet und mittels einer aufschraubbaren Überwurfmutter 97 daran auswechselbar befestigt. Wie weiterhin in Fig.1A dargestellt umfasst die erste Funktionseinheit 90 eine Führungshülse 91, eine röhrchenförmige Sonde 96 sowie das schematisch dargestellte Klemmelement 95. Die Funktionseinheit 90 wird später in Verbindung mit den Figuren 11A und 11B noch im einzelnen beschrieben.

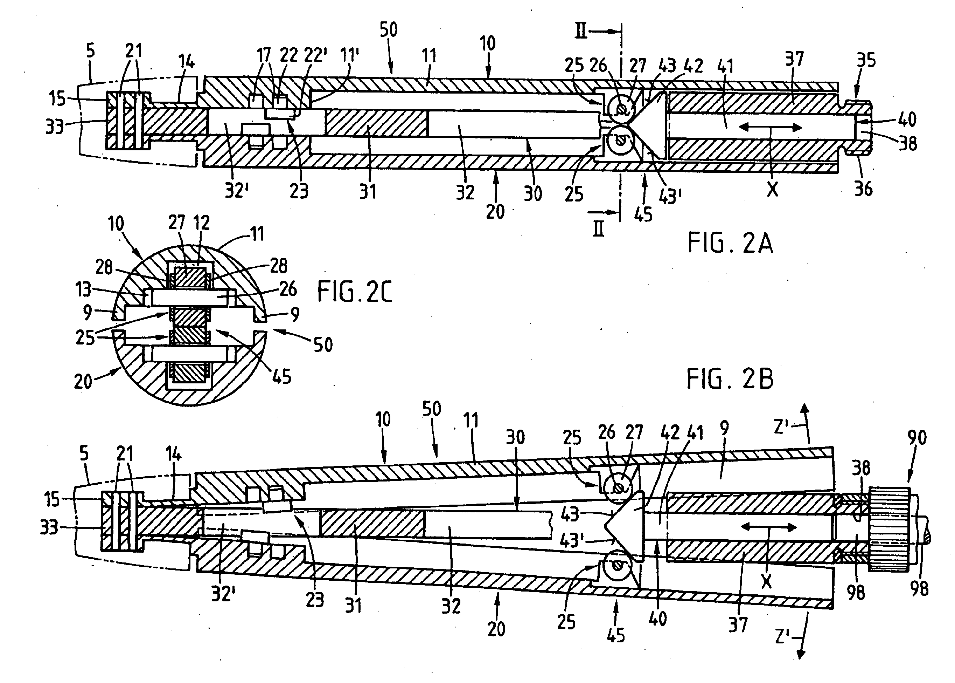

Im Bereich der Verschlusskappe 5 sind die beiden Gehäuseteile 10 und 20 in Form eines theoretischen Drehpunktes (Fig.2A, 2B) derart miteinander verbunden, dass diese jeweils entgegen einer federelastisch wirkenden Rückstellkraft sowie bei montierter Funktionseinheit 90 (Fig.1A und 1C) am vorderen Ende relativ zueinander aufgeschwenkt beziehungsweise gespreizt zueinander angeordnet sind. Durch Zusammendrücken der beiden mit einer in den Figuren 1A und 1C schematisch dargestellten ersten Übertragungsvorrichtung 45 wirkverbundenen Gehäuseteile 10 und 20 gemäss Pfeilrichtung Z wird das in Fig.1A schematisch dargestellte Klemmelement 95 betätigt.

In Fig.1B ist das chirurgische Instrument 150 in schematischer Ansicht dargestellt und man erkennt das mit der teilweise dargestellten Verschlusskappe 5 und den beiden Gehäuseteilen 10 und 20 versehene Gehäuse 50, das an dem Tragarm 30 angeordnete und mit einem Aussengewinde 36 versehene Kopfstück 35 sowie die in Bezug dazu demontiert dargestellte erste Funktionseinheit 90. Wie vorstehend erwähnt, umfasst die erste Funktionseinheit 90 die Führungshülse 91, die mit einem Stössel 98 wirkverbundene Sonde 96, eine in der Führungshülse 91 angeordnete Druckfeder 93 und eine Stange 92, welche am vorderen distalen Ende mit dem Klemmelement 95 (Fig.1A) versehen ist.

Der in demontiertem Zustand teilweise aus der Funktionseinheit 90 herausragende Stössel 98 steht in zusammengebautem Zustand mit einem Stellglied 40 (Fig.2B) der ersten Übertragungsvorrichtung 45 in Wirkverbindung. Mit dem anderen Ende ist der Stössel 98 derart mit der koaxial in der Führungshülse 91 angeordneten Sonde 96 wirkverbunden, dass beim Zusammendrücken der beiden Gehäuseteile 10 und 20 gemäss Pfeilrichtung Z (Fig.1A) der Stössel 98 zusammen mit der wirkverbundenen Sonde 96 entgegen der Rückstellkraft der Druckfeder 93 in axialer Richtung verschoben und dabei das Klemmelement 95 betätigt wird. Die einzelnen Elemente der ersten Funktionseinheit 90 sowie die Wirkungsweise derselben wird in Verbindung mit den Figuren 11A und 11B noch im einzelnen beschrieben.

In Fig.1C ist das chirurgische Instrument 150 mit dem Gehäuse 50 und den beiden Gehäuseteilen 10 und 20 sowie die am Kopfstück 35 angeschraubte erste Funktionseinheit 90 in Ansicht dargestellt. Die beiden Gehäuseteile 10 und 20 sind infolge der angeschraubten und montierten Funktionseinheit 90 um den im Bereich der Verschlusskappe 5 angeordneten theoretischen Drehpunkt gemäss Pfeilrichtung Z' aufgeschwenkt beziehungsweise relativ zueinander gespreizt. Zwischen den beiden Gehäuseteilen 10 und 20 ist der mit dem Kopfstück 35 versehene Tragarm 30 für die Funktionseinheit 90 sowie die hier schematisch dargestellte erste Übertragungsvorrichtung 45 angeordnet.

Fig.1D zeigt ein Teilstück der in schematischer Ansicht dargestellten Führungshülse 91, die darin angeordnete Sonde 96 sowie die koaxial darin angeordnete und in axialer Richtung orientierte Stange 92. Am vorderen distalen Ende ist die Stange 92 mit zwei gespreizt zueinander angeordneten Klemmarmen 94.1 und 94.2 versehen, welche das mit 95 bezeichnete Klemmelement bilden. Bei der in Fig.1A bis 1D dargestellten ersten Variante wird beim Zusammendrücken (Fig.1A) der beiden Gehäusehälften 10 und 20 Stössel 98 zusammen mit der röhrchenförmigen Sonde 96 entgegen der Rückstellkraft der Druckfeder 93 axial in Pfeilrichtung X' (Fig.1D) sowie relativ zu der Stange 92 verschoben und dabei zur Erreichung der schematisch dargestellten Klemmfunktion (Fig.1E) über die beiden Klemmarme 94.1 und 94.2 geschoben. Durch Loslassen der beiden Gehäuseteile 10 und 20 werden diese gemäss Pfeilrichtung Z' (Fig.1C) selbsttätig geöffnet und infolge der Rückstellkraft der Druckfeder 93 die röhrchenförmige Sonde 96 relativ zu den beiden Klemmarmen 94.1 und 94.2 gemäss Pfeilrichtung X" (Fig.1E) in die Führungshülse 91 zurückgezogen.

Die vorstehend beschriebene erste Funktionseinheit 90 hat den Vorteil, dass der für den chirurgischen Eingriff mittels der beiden Klemmarme 94.1 und 94.2 bewirkte und in Fig.1E schematisch dargestellte Klemmpunkt in Bezug auf die Stirnseite der Führungshülse 91 örtlich stets stationär ist. Bei einer nicht näher dargestellten Variante besteht jedoch auch die Möglichkeit, dass bei Betätigung der beiden Gehäuseteile 10 und 20 (Fig.1A) die Stange 92 mit den beiden angeformten Klemmarmen 94.1 und 94.2 zur Erreichung der Klemmfunktion in die röhrchenförmige Sonde 96 eingezogen und infolge dessen ein in axialer Richtung und in Bezug auf die Stirnseite der Führungshülse 91 örtlich variabler Klemmpunkt erreicht wird. Bei dieser Variante ist die Sonde 96 fest an der Führungshülse 91 angeordnet und die Stange 92 mit den beiden angeformten Klemmarmen 94.1 und 94.2 relativ zu dem vorderen Ende der Sonde 96 in axialer Richtung verschiebbar.

Fig.1F zeigt als weitere Variante eine teilweise dargestellte zweite Funktionseinheit 90', welche abweichend von der Variante gemäss Fig.1D am vorderen Ende mit einem Schneidelement 105 versehen ist. Die zweite Funktionseinheit 90' umfasst eine Führungshülse 110, eine daran angeordnete röhrchenförmige Sonde 106 sowie eine koaxial darin angeordnete Stange 109. Die Stange 109 ist über einen Gewindestift 111 mit einem Wendebolzen (Fig.11C) wirkverbunden. Das Schneidelement 105 umfasst ein erstes und zweites Schneidblatt 107 und 108, wobei das erste Schneidblatt 107 am vorderen Ende der röhrchenförmigen Sonde 106 und das zweite Schneidblatt 108 am vorderen Ende der Stange 109 angeformt ist. Zur Erreichung der Schneidfunktion wird das an der Stange 109 angeformte zweite Schneidblatt 108 infolge einer um die Längsachse 109' orientierten Drehbewegung mit dem feststehenden Schneidblatt 107 in Eingriff gebracht. Die einzelnen Elemente der zweiten Funktionseinheit 90' sowie die Wirkungsweise derselben wird in Verbindung mit Fig.11C im einzelnen beschrieben.

Fig.1G zeigt das in Ansicht sowie in grösserem Massstab dargestellte Schneidelement 105 mit den beiden Schneidblättern 107 und 108, wobei das an der Stange 109 angeordnete zweite Schneidblatt 108 relativ zu dem an der Sonde 106 angeordneten ersten Schneidblatt 107 in Pfeilrichtung Y' schwenkbar ist. Bei dieser Variante erfolgt die Schneidfunktion unter Beibehaltung einer in axialer Richtung örtlich stationären Schneidstelle durch die um die Längsachse 109' der Stange 109 orientierte Schwenkbewegung des zweiten Schneidblattes 108 relativ zu dem feststehenden ersten Schneidblatt 107.

Die spezielle Ausgestaltung der beiden in den Figuren 1D bis 1G dargestellten Varianten des Klemmelements 95 mit den beiden Klemmarmen 94.1 und 94.2 beziehungsweise des Schneidelements 105 mit den beiden Schneidblättern 107 und 108 sind nicht Gegenstand dieser Erfindung und sind folglich auch nicht näher beschrieben.

In Fig.2A ist das Gehäuse 50 mit den beiden Gehäuseteilen 10 und 20 und dem dazwischen angeordneten Tragarm 30 sowie der daran angeordneten ersten Übertragungsvorrichtung 45 in grösserem Massstab sowie in geschlossener Stellung dargestellt. Fig.2B zeigt das Gehäuse 50, bei welchem die Gehäuseteile 10 und 20 infolge der montierten und in Fig.2B nur teilweise dargestellten Funktionseinheit 90 gemäss Pfeilrichtung Z' aufgeschwenkt beziehungsweise in Bezug auf den Tragarm 30 in gespreizter Stellung dargestellt sind. Die einzelnen Elemente 10,20,30 und 45 werden nachstehend in Verbindung mit Figur 2A und Figur 2B beschrieben.

Das im Schnitt dargestellte erste Gehäuseteil 10 umfasst eine in Längsrichtung orientierte und im Profilquerschnitt etwa kreisbogenförmig (Fig.2C) ausgebildete Gehäusewand 11. Die Gehäusewand 11 hat am hinteren proximalen Ende ein abgesetzt ausgebildetes Wandstück 11' sowie ein daran angeformtes Endstück 15. Zwischen dem abgesetzten Wandstück 11' und dem Endstück 15 ist ein etwa plättchenförmig ausgebildeter und die beiden Teile 11' und 15 miteinander verbindender Federarm 14 angeformt. Der Federarm 14 bildet im wesentlichen den vorstehend erwähnten, nicht näher dargestellten theoretischen Drehpunkt der beiden Gehäuseteile 10 un 20. An dem vorderen Ende der Gehäuseteile 10, 20 ist jeweils an der Innenseite der Gehäusewand 11 eine Lagerung 25 zur Aufnahme eines Gleitelements angeordnet. Im dargestellten Ausführungsbeispiel umfasst die Lagerung 25 eine an einer Achse 26 drehbar gelagerte Laufrolle 27.

Der im Schnitt dargestellte und etwa als flache längliche Schiene ausgebildete Tragarm 30 hat am vorderen Ende ein mit dem einstückig angeformten Kopfstück 35 versehenes zylindrisches Führungsgehäuse 37. Am hinteren Ende ist der Tragarm 30 mit einem Auflageteil 33 versehen, an welchem der Federarm 14 sowie das Endstück 15 der Gehäuseteile 10 und 20 flach anliegend angeordnet sind. Der Tragarm 30 ist zwischen dem Führungsgehäuse 37 und dem Auflageteil 33 mit einer ersten Ausnehmung 32, einem Steg 31 sowie mit einer zweiten Ausnehmung 32' versehen. Das Führungsgehäuse 37 sowie das daran einstückig angeformte und mit einem Aussengewinde 36 versehene Kopfstück 35 werden von einer in axialer Richtung orientierten Bohrung 38 durchdrungen. Die Bohrung 38 ist zur Lagerung des Stellgliedes 40 ausgebildet und steht mit der ersten Ausnehmung 32 des Tragarms 30 in Verbindung. Das Stellglied 40 hat einen in der Bohrung 38 angeordneten Bolzen 41 sowie ein daran angeformtes und in der Ausnehmung 32 des Tragarms 30 geführtes Gleitstück 42. Auf der dem Bolzen 41 abgewandten Seite ist das Gleitstück 42 mit zwei keilförmigen Gleitflächen 43 und 43' versehen.

In Fig.2B ist als Ausführungsbeispiel ein Teilstück der ersten Funktionseinheit 90 dargestellt, welche mit dem Stössel 98 in der Bohrung 38 des Kopfstücks 35 beziehungweise des Führungsgehäuses 37 angeordnet ist und mit dem Bolzen 41 des Stellgliedes 40 zusammenwirkend in Eingriff steht. Es besteht jedoch auch die Möglichkeit, dass anstelle der ersten Funktionseinheit 90 die zweite Funktionseinheit 90' am Kopfstück 35 angeschraubt wird.

Bei der in axialer Richtung orientierten Bewegung des Stellgliedes 40 ist dieses mit dem Gleitstück 42 in der ersten Ausnehmung 32 des Tragarms 30 exakt geführt und gegen Verdrehung gesichert. Die am Gleitstück 42 angeformten Gleitflächen 43 und 43' stehen dabei mit den beiden an den Gehäuseteilen 10 und 20 gelagerten und korrespondierend zueinander angeordneten Laufrollen 27 in Eingriff. Ausgehend von der in Fig.2A dargestellten Stellung werden bei der in axialer Richtung orientierten Bewegung des Stellgliedes 40 die beiden Gehäuseteile 10 und 20 entgegen der federelastischen Rückstellkraft des am hinteren Ende der Gehäuseteile 10 und 20 vorgesehenen Federarms 14 gemäss Pfeilrichtung Z' aufgeschwenkt beziehungsweise relativ zu dem Tragarm 30 gespreizt (Fig.2B).

Wie in Fig.2A und Fig.2B weiterhin dargestellt, sind die beiden Gehäuseteile 10 und 20 sowie der mit dem Auflageteil 33 dazwischen angeordnete Tragarm 30 an dem hinteren proximalen Ende durch zwei im Abstand zueinander angeordnete Stifte 21 sowie durch die aufschiebbare Verschlusskappe 5 miteinander verbunden. In dem abgesetzt ausgebildeten Wandstück 11' der beiden Gehäuseteile 10 und 20 ist jeweils ein mit einem Zapfen 22 und einem kopfförmigen Absatz 22'versehenes Sperrglied 23 in einer Ausnehmung 17 angeordnet und mit nicht dargestellten Mitteln befestigt. Die versetzt zueinander in den Ausnehmungen 17 der beiden Gehäuseteile 10 und 20 angeordneten Sperrglieder 23 sind zur Erreichung einer Bewegungsbegrenzung der beiden Gehäuseteile 10,20 mit dem entsprechend ausgebildeten Absatz 22' in der zweiten Ausnehmung 32' des Tragarms 30 angeordnet.

Fig.2C zeigt das gemäss der in Fig.2A eingezeichneten Linie II-II im Schnitt dargestellte Gehäuse 50 und man erkennt die beiden Gehäuseteile 10 und 20 mit der jeweils daran angeordneten Lagerung 25 für die erste Übertragungsvorrichtung 45. An der Innenseite der Gehäusewand 11 ist eine erste Ausnehmung 12 für die an der Achse 26 drehbar gelagerte Laufrolle 27 angeordnet. An beiden Seiten der Laufrolle 27 ist jeweils eine Gleitscheibe 28 angeordnet und mit nicht dargestellten Mitteln befestigt. Die Achse 26 ist in einer zwischen den beiden seitlichen Wänden 9 des Gehäuses 10 vorgesehenen zweiten Ausnehmung 13 gelagert. Die beiden Gehäuseteile 10 und 20 sind, wie in Fig.2C dargestellt symmetrisch ausgebildet, weshalb vorstehend nur das eine Gehäuseteil 10 beschrieben und mit Bezugszeichen versehen ist.

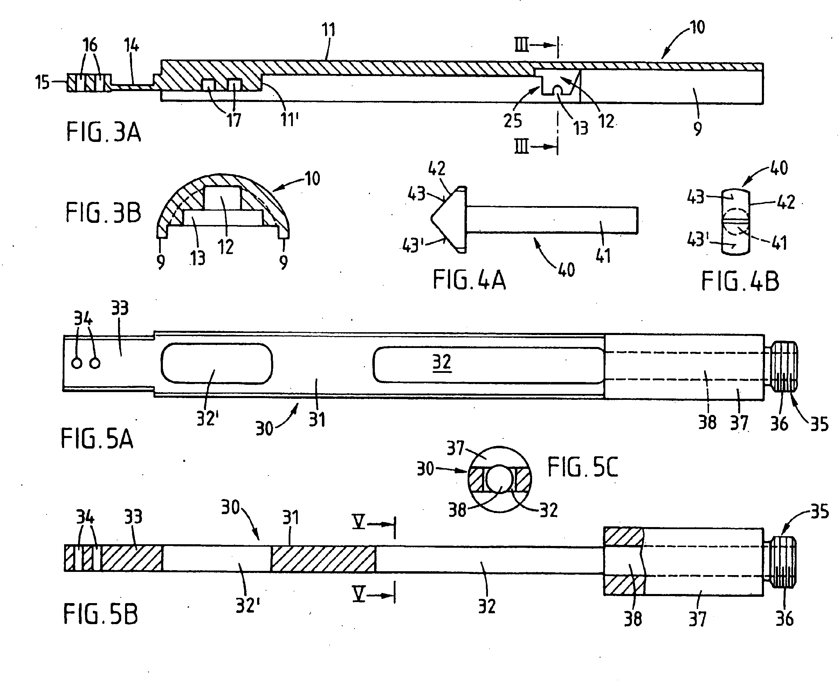

In Verbindung mit den Figuren 3 bis 5 werden nachstehend die Funktionselemente des Instruments 150 im einzelnen beschrieben.

In Fig.3A ist das erste Gehäuseteil 10 im Schnitt dargestellt und man erkennt die in Längsrichtung orientierte Gehäusewand 11 mit den beiden seitlichen Wänden 9, das am hinteren proximalen Ende daran angeformte und abgesetzt ausgebildete Wandstück 11', den etwa plättchenförmig ausgebildeten Federarm 14 sowie das daran angeformte Endstück 15. In dem Endstück 15 sind zwei im Abstand zueinander angeordnete Bohrungen 16 für die Stifte 21 (Fig.2A und Fig.2B) vorgesehen. In dem Wandteil 11' sind zwei im Abstand zueinander angeordnete Sacklochbohrungen 17 zur Aufnahme und Befestigung des in Fig.2A und 2B dargestellten Rastteils 23 angeordnet. Am vorderen Ende des Gehäuseteils 10 ist an der Innenseite die erste Ausnehmung 12 für die Laufrolle 27 sowie die zweite Ausnehmung 13 für die Achse 26 angeordnet. Die zweite Ausnehmung 13 ist vorzugsweise so ausgebildet, dass die Achse 26 klemmend darin gehalten ist. In Fig.3B ist das Gehäuseteil 10 gemäss der in Fig.3A eingezeichneten Linie III-III im Profilquerschnitt dargestellt und man erkennt die nutförmige erste Ausnehmung 12 sowie die zwischen den beiden seitlichen, stegförmigen Wänden 9 angeordnete zweite Ausnehmung 13.

In Fig. 4A ist das Stellglied 40 für die erste Übertragungsvorrichtung 45 in Ansicht und in Fig. 4B in Seitenansicht dargestellt und man erkennt den länglichen Bolzen 41 sowie das daran angeformte Gleitstück 42. Das mit den beiden parallel zueinander angeordneten und nicht höher bezeichneten Seitenwänden (Fig. 4B) versehene Gleitstück 42 ist auf der dem Bebolzen 41 abgewandten Seite mit den beiden keilförmigen Gleitflächen 43 und 43' oder Gleitkufen versehen.

Bei dem in Fig.4A dargestellten Ausführungsbeispiel sind die beiden Gleitflächen 43 und 43' des Gleitstücks 42 als gerade (ebene) und in Richtung der nicht bezeichneten Spitze geneigte Flächen ausgebildet. Bei einer weiteren, nicht näher dargestellten Variante besteht jedoch auch die Möglichkeit, dass die beiden Gleitflächen 43 und 43' im Profilquerschnitt (nicht dargestellt) konkav oder konvex ausgebildet sind. Mit dieser Formgebung wird eine progressive beziehungsweise degressive Abrollbewegung der beiden mit den Gleitflächen 43 und 43' in Eingriff stehenden Rollen 27 (Fig.2A und 2B) erreicht.

In Fig.5A ist der als längliche Schiene ausgebildete Tragarm 30 in Draufsicht und in Fig.5B im Schnitt dargestellt und man erkennt das mit dem einstückig angeformten Kopfstück 35 versehene Führungsgehäuse 37, die erste Ausnehmung 32, den Steg 31 und die zweite Ausnehmung 32' mit dem angeformten Auflageteil 33. Die in axialer Richtung das Führungsgehäuse 37 sowie das mit dem Aussengewinde 36 versehene Kopfstück 35 durchdringende Bohrung 38 mündet in der zugeordneten ersten Ausnehmung 32. Das am hinteren Ende des Tragarms 30 angeordnete Auflageteil 33 wird von zwei im Abstand zueinander angeordneten Bohrungen 34 für die in Fig.2A und Fig.2B dargestellten Stifte 21 durchdrungen. Fig.5C zeigt den gemäss der in Fig.5B eingezeichneten Linie V-V im Schnitt dargestellten Tragarm 30 und man erkennt das Gehäuse 37 mit der Bohrung 38 sowie die Ausnehmung 32 des Tragarms 30.

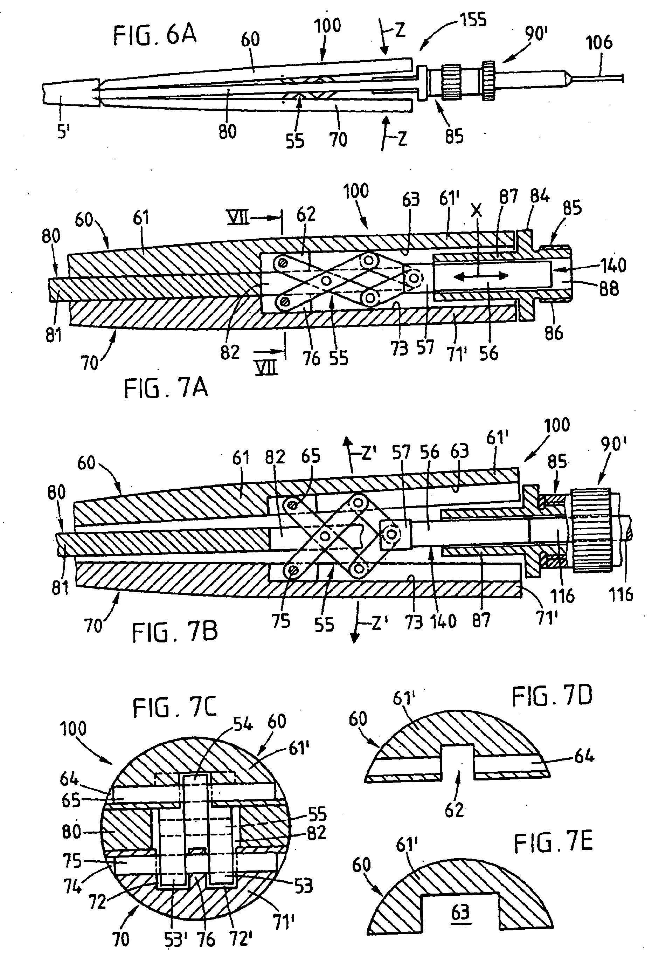

Fig.6A zeigt als zweites Ausführungsbeispiel ein in schematischer Ansicht dargestelltes und in der Gesamtheit mit 155 bezeichnetes chirurgisches Instrument zur Durchführung ophthalmologischer Eingriffe. Dieses zweite Ausfürungsbeispiel ist nicht Gegenstand der Schutzansprüche. Das Instrument 155 umfasst ein als Handgriff ausgebildetes und mit zwei Gehäuseteilen 60 und 70 sowie einem dazwischen angeordneten Tragarm 80 versehenes Gehäuse 100. An dem hinteren Ende des Gehäuses 100 sind die Teile 60,70 und 80 vorzugsweise analog den vorstehend in Verbindung mit Fig.2A und 2B beschriebenen Teilen 10,20 und 30 ausgebildet und mit einer aufsteckbaren Verschlusskappe 5' versehen.

An dem vorderen Ende des zwischen den beiden Gehäuseteilen 60 und 70 angeordneten Tragarms 80 ist an einem Kopfstück 85 entweder die erste Funktionseinheit 90 (Fig.1D) oder die zweite Funktionseinheit 90' (Fig.1F) angeordnet. Die beiden Funktionseinheiten 90 und 90' werden nachstehend in Verbindung mit den Figuren 11A bis 11C beschrieben. Zwischen den beiden in Fig.6A relativ zueinander aufgeschwenkt beziehungsweise gespreizt dargestellten Gehäuseteilen 60 und 70 ist der mit dem Kopfstück 85 versehene Tragarm 80 für eine mit der Funktionseinheit 90 oder 90' wirkverbundene und in Fig.6A schematisch dargestellte zweite Übertragungsvorrichtung 55 angeordnet. Durch Zusammendrücken der beiden Gehäuseteile 60 und 70 gemäss der in Fig.6A eingezeichneten Pfeilrichtung Z wird mittels der zweiten Übertragungsvorrichtung 55 und einem damit wirkverbundenen Stellglied 140 und dem Stössel 98 (Fig.7B) das in der Sonde 96 angeordnete Klemmelement 95 betätigt. Das Schneidelement 105 wird von einem weiteren Stellglied 116 gemäss Fig.11C betätigt.

In Fig.7A ist das Gehäuse 100 mit den beiden Gehäuseteilen 60 und 70 sowie der dazwischen angeordnete Tragarm 80 mit der zweiten Übertragungsvorrichtung 55 in grösserem Massstab und in geschlossener Stellung dargestellt. Fig.7B zeigt das Gehäuse 100, bei welchem die beiden Gehäuseteile 60 und 70 infolge der montierten und in Fig.7B nur teilweise dargestellten Funktionseinheit 90 oder 90' gemäss Pfeilrichtung Z' aufgeschwenkt beziehungsweise in gespreizter Stellung dargestellt sind. Die einzelnen Elemente 60,70 und 80 sowie 55 und 140 werden nachstehend beschrieben.

Das gemäss Fig.7A im Schnitt dargestellte erste Gehäuseteil 60 hat eine Wand 61, welche im Profilquerschnitt etwa in Form eines Kreisbogensegments (Fig.7D und 7E) ausgebildet ist. Im Bereich des vorderen Teilstücks 61' ist an der nicht bezeichneten Innenseite eine erste nutförmige Aussparung 62 sowie eine daran anschliessende und in axialer Richtung orientierte erste Führungsnut 63 vorgesehen. Das zweite Gehäuseteil 70 hat eine Wand 71, welche im Bereich des vorderen Teilstücks 71' mit zwei durch einen Steg 76 voneinander getrennten Aussparungen 72,72' (Fig.7C) sowie mit einer daran anschliessenden und in axialer Richtung orientierten zweiten Führungsnut 73 versehen ist.

Weiterhin erkennt man in Fig.7A die etwa als Scherengitter ausgebildete und in Längsrichtung in einer Ausnehmung 82 des Tragarms 80 geführte zweite übertragungsvorrichtung 55. Die zweite Übertragungsvorrichtung 55 ist mit dem einen Ende an den beiden Gehäuseteilen 60 und 70 und mit dem anderen Ende an dem Stellglied 140 angelenkt. Das mit der zweiten Übertragungsvorrichtung 55 wirkverbundene Stellglied 140 hat einen etwa gabelförmig ausgebildeten Halter 57 sowie einen daran befestigten länglichen Bolzen 56. Der längliche Bolzen 56 ist in einer Bohrung 88 des mit dem Kopfstück 85 versehenen Führungsgehäuses 87 angeordnet und bei der in Doppelpfeilrichtung X orientierten Bewegung der zweiten Übertragungsvorrichtung 55 verschiebbar.

Der in Fig.7A und Fig.7B im Schnitt dargestellte und als flache längliche Schiene ausgebildete Tragarm 80 ist zwischen dem Führungsgehäuse 87 und dem ersten Steg 81 mit der ersten Ausnehmung 82 versehen. In der Ausnehmung 82 ist die mit mehreren gelenkig miteinander verbundenen Laschen versehene zweite Übertragungsvorrichtung 55 sowie das damit wirkverbundene Stellglied 140 bei den in Pfeilrichtung Z oder Z' orientierten Bewegungen der beiden Gehäuseteile 60 und 70 exakt geführt.

Die beiden Gehäuseteile 60 und 70 sowie der dazwischen angeordnete Tragarm 80 sind analog, wie vorstehend in Verbindung mit den Figuren 2A und 2B beschrieben, an dem hinteren Ende miteinander verbunden. Bei montierter Funktionseinheit 90 oder 90' mit dem in der Bohrung 88 angeordneten Stössel 98 oder 116 werden die beiden Gehäuseteile 60 und 70 entgegen der eigenen federelastisch wirkenden Rückstellkraft, wie in Fig.7B dargestellt, relativ zueinander sowie zu dem Tragarm 80 aufgeschwenkt beziehungsweise gespreizt. Durch das Zusammendrücken der beiden Gehäuseteile 60 und 70 gemäss Pfeilrichtung Z (Fig.6A) wird mittels der scherengitterförmigen Übertragungsvorrichtung 55 das Stellglied 140 mit dem Stössel 98 oder 116 in axialer Richtung verschoben und dabei das in der Sonde 96 angeordnete und in Fig.7A und 7B nicht dargestellte Klemmelement 95 (Fig.1D) oder das Schneidelement 105 (Fig.1F) entsprechend betätigt.

Fig.7C zeigt dass gemäss der in Fig.7A eingezeichneten Linie VII-VII im Schnitt dargestellte Gehäuse 100 und man erkennt die beiden Gehäuseteile 60 und 70 mit dem dazwischen angeordneten Tragarm 80 sowie die in der Ausnehmung 82 desselben angeordnete zweite Übertragungsvorrichtung 55. Die aus mehreren, gelenkig miteinander verbundenen Laschen etwa als Scherengitter ausgebildetezweite Übertragungsvorrichtung 55 ist mit einer ersten Lasche 54 in der nutförmigen Aussparung 62 des ersten Gehäuseteils 60 angeordnet und an einem Bolzen 65 gelagert. Weiterhin ist die zweite Übertragungsvorrichtung 55 mit zwei Laschen 53 und 53' in den Aussparungen 72 und 72' des zweiten Gehäuseteils 70 angeordnet und mit einem Bolzen 75 an einem Steg 76 gelagert.

Fig.7D zeigt das im Schnitt sowie in grösserem Masstab dargestellte erste Gehäuseteil 60 mit der in dem Teilstück 61' angeordneten, nutförmigen Aussparung 62 für die Lagerung der zweiten Übertragungsvorrichtung 55. Das Gehäuseteil 60 ist weiterhin mit einer quer zu der Aussparung 62 angeordneten Bohrung 64 versehen, in welcher der zur Lagerung der ersten Lasche 54 vorgesehene Bolzen 65 einsetzbar ist (Fig.7C). In Fig.7E ist das erste Gehäuseteil 60 mit der in dem Teilstück 61' angeordneten Führungsbahn 63 für zwei nebeneinander angeordnete Laschen der zweiten Übertragungsvorrichtung 55 im Profilquerschnitt dargestellt.

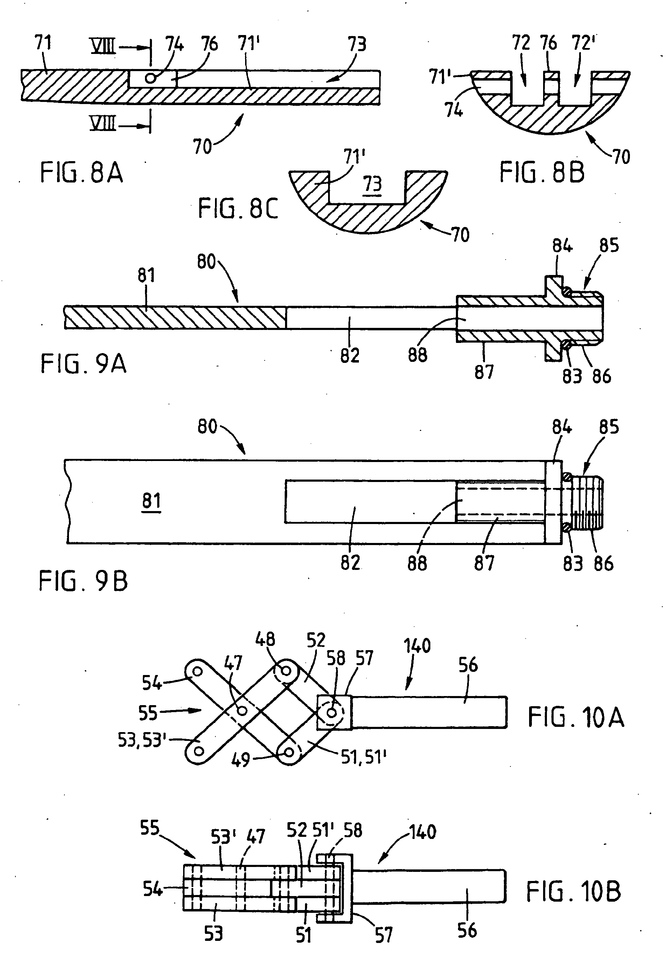

In Fig.8A ist ein Teilstück des zweiten Gehäuseteils 70 im Schnitt dargestellt und man erkennt die Wand 71 sowie das abgesetzt dazu ausgebildete vordere Teilstück 71' mit dem an der Innenseite angeordneten Steg 76. Fig.8B zeigt das im Profilquerschnitt gemäss der Linie VIII-VIII dargestellte zweite Gehäuseteil 70 mit dem Steg 76 und den beiden nutförmigen Aussparungen 72 und 72' für die Lagerung der zweiten Übertragungsvorrichtung 55. Das Gehäuseteil 70 ist weiterhin mit einer quer zu den Aussparungen 72 und 72' angeordneten und den Steg 76 durchdringenden Bohrung 74 versehen, in welcher der zur Lagerung der beiden Laschen 53,53' vorgesehene Bolzen 75 einsetzbar ist. In Fig.8C ist das Gehäuseteil 70 mit der in dem Teilstück 71' angeordneten Führungsbahn 73 für zwei nebeneinander angeordnete Laschen der zweiten Übertragungsvorrichtung 55 im Profilquerschnitt dargestellt.

Fig.9A zeigt ein im Schnitt dargestelltes Teilstück des als längliche Schiene ausgebildeten Tragarms 80, welcher in Fig.9B in Draufsicht dargestellt ist. Der Tragarm 80 umfasst das mit dem einstückig angeformten Kopfstück 85 versehene Führungsgehäuse 87 mit der Bohrung 88 sowie die damit verbundene Ausnehmung 82 und den Steg 81. An dem mit dem Aussengewinde 86 versehenen Kopfstück 85 des Führungsgehäuses 87 ist zur abdichtenden Anlage der Funktionseinheit 90 oder 90' ein Flansch 84 mit einer Dichtung 83 angeordnet.

Fig.10A zeigt die in Ansicht dargestellte zweite Übertragungsvorrichtung 55 mit dem daran angeordneten Stellglied 140 für das in Fig.6A beziehungsweise Fig.7A und 7B schematisch und in grösserem Massstab dargestellte chirurgische Instrument 155. Die zweite Übertragungsvorrichtung 55 umfasst den dem Bolzen 56 befestigten und etwa ]-förmig ausgebildeten Halter 57. An den beiden nicht bezeichneten seitlichen Stegen ist der Halter 57 mit entsprechenden Bohrungen versehen, in welchen ein Lagerbolzen 58 angeordnet ist. An dem Lagerbolzen 58 ist eine erste Lasche 52 und zu beiden Seiten derselben jeweils eine zweite Lasche 51 und 51' gelagert. An dem anderen Ende der ersten Lasche 52 sind zu beiden Seiten derselben an einem Bolzen 48 dritte Laschen 53 und 53' angelenkt. An dem freien Ende der Laschen 51 und 51' ist eine vierte Lasche 54 angelenkt. Die dritten Laschen 53, 53' sowie die dazwischen angeordnete vierte Lasche 54 sind weiterhin durch einen Bolzen 47 miteinander verbunden.

Die in Form eines Scherengitters ausgebildete zweite Übertragungsvorrichtung 55 ist einerseits mit den ersten und zweiten Laschen 51,51' und 52 an dem mit dem Bolzen 56 versehenen Halter 57 des Stellgliedes 140 angelenkt und andererseits mit den dritten Laschen 53,53' in den Aussparungen 72,72' des zweiten Gehäuseteils 70 sowie mit der vierten Lasche 54 in der Aussparung 62 des ersten Gehäuseteils 60 angeordnet und gelagert.

In Fig.10B ist die zweite Übertragungsvorrichtung 55 mit dem Stellglied 140 in Draufsicht dargestellt und man erkennt den etwa ]-förmig ausgebildeten Halter 57 mit dem Bolzen 56 sowie die einzelnen im wesentlichen an dem Halter 57 gelagerten Laschen 51,51' und 52 sowie die daran gelagerten Laschen 53,53' und 54.

In Fig.11A ist die in Fig.1B schematisch dargestellte erste Funktionseinheit 90 im Schnitt sowie in grösserem Massstab dargestellt und man erkennt die mit einer Ausnehmung 97.1 versehene Überwurfmutter 97, die koaxial darin angeordnete Führungshülse 91, einen daran angeordneten Zwischenring 97.2 und einen Stellring 97.3. Der Stellring 97.3 ist mit einem Gewindestift 97.4 an der Führungshülse 91 derart fixiert, dass die Überwurfmutter 97 und die Führungshülse 91 eine Baueinheit bilden. Weiterhin erkennt man das koaxial in einer in axialer Richtung orientierten Ausnehmung 91.2 der Führungshülse 91 angeordnete Stössel 98. Der Stössel 98 ist an dem einen Ende mit einem angeformten Absatz 98.3 versehen, auf welchem die mit dem anderen Ende an der Innenseite der Ausnehmung 91.2 der Führungshülse 91 abgestützte Druckfeder 93 gelagert ist. Der Stössel 98 ist weiterhin mit einer in axialer Richtung orientierten Sacklochbohrung 98.1 versehen, welche zur Aufnahme der bis zu einer Anlagekante 98.4 eingeschoben und beispielsweise in Form einer Kleb- oder Schweissverbindung damit wirkverbundenen röhrchenförmigen Sonde 96 ausgebildet ist. In der röhrchenförmigen Sonde 96 ist koaxial die in axialer Richtung orientierte Stange 92 angeordnet. Die Stange 92 ist an dem einen Ende durch mindestens einen quer zur axialen Richtung in die Führungshülse 91 eingeschraubten Gewindestift 99 oder dergleichen gegen axiales Verschieben gesichert und fixiert. An dem anderen Ende der aus der röhrchenförmigen Sonde 96 ragenden Stange 92 ist diese mit den zwei angeformten und relativ zueinander aufgebogenen Klemmarmen 94.1 und 94.2 versehen, welche zusammen das Klemmelement 95 bilden.

In Fig.11B ist das mit einer in axialer Richtung orientierten Ausnehmung 98.2 für den Gewindestift 99 versehene Stössel 98 zusammen mit der röhrchenförmigen Sonde 96 entgegen der Rückstellkraft der Druckfeder 93 in axialer Richtung relativ zu der durch den Gewindestift 99 gesicherten Stange 92 derart gemäss Pfeilrichtung X' verschoben, dass die beiden Klemmarme 94.1 und 94.2 mittels der darüber geschobenen röhrchenförmigen Sonde 96 zusammengedrückt werden und dabei die Klemmfunktion bewirken.

In Fig.11C ist die in Fig.1C schematisch dargestellte zweite Funktionseinheit 90' im Schnitt sowie in grösserem Massstab dargestellt und man erkennt eine Überwurfmutter 115 mit einer Ausnehmung 115.1 und eine koaxial darin angeordnete Führungshülse 110. An der Führungshülse 110 ist ein Zwischenring 115.2 sowie ein Stellring 115.3 angeordnet, welcher mit einem Gewindestift 115.4 derart an der Führungshülse 110 fixiert ist, dass die Überwurfmutter 115 und die Führungshülse 110 eine Baueinheit bilden. Die Führungshülse 110 ist mit einer in axialer Richtung orientierten Ausnehmung 110.1 versehen, welche zur Aufnahme des mit einer Sacklochbohrung 116.1 versehenen Stössels 116 ausgebildet ist. Weiterhin ist in der Ausnehmung 110.1 der Führungshülse 110 ein Wendebolzen 114 sowie eine Scheibe 112 angeordnet. Der Wendebolzen 114 ist an dem einen Ende mit einem abgesetzt ausgebildeten Teilstück 114.1 koaxial in der Sacklochbohrung 116.1 des Stössels 116 angeordnet. An dem abgesetzten Teilstück 114.1 ist eine an dem Stössel 116 sowie am Wendebolzen 114 abgestützte Druckfeder 113 angeordnet. Im Bereich der Sacklochbohrung ist der Stössel 116 mit einer kulissenförmigen Ausnehmung 116.2 versehen, in welcher ein an dem Teilstück 114.1 des Wendebolzens 114 befestigter Stift 114.2 geführt ist. An dem anderen Ende des Wendebolzens 114 ist die mit dem angeformten zweiten Schneidblatt 108 versehene Stange 109 angeordnet. Die Stange 109 und der Wendebolzen 114 sind durch einen einschraubbaren Gewindestift 111 miteinander wirkverbunden. Weiterhin erkennt man in Fig.11C die röhrchenförmige Sonde 106, welche an dem distalen Ende das angeformte erste Schneidblatt 107 und mit dem anderen Ende in die Führungshülse 110 eingesteckt und befestigt ist.

Bei der in Fig.11C dargestellten zweiten Funktionseinheit 90' wird bei der in axialer Richtung entgegen der Rückstellkraft der Druckfeder 113 orientierten Bewegung X' des Stössels 116 sowie der damit wirkverbundene Wendebolzen 114 mit der Stange 109 infolge des in der kulissenförmigen Ausnehmung 116.2 geführten Stiftes 114.2 zwangsläufig um die theoretische Längsachse 109' gedreht. Mit der um die Längsachse 109' orientierten Drehbewegung der Stange 109 wird gleichzeitig das daran angeformte zweite Schneidblatt 108 gemäss der in Fig.1G eingezeichneten Pfeilrichtung Y' mit dem feststehenden ersten Schneidblatt 107 für den Schneidvorgang in Eingriff gebracht.

## Patentansprüche

1. Chirurgisches Instrument, insbesondere zur Durchführung ophthalmologischer Eingriffe, bestehend aus einem Gehäuse (50) mit zwei länglichen Gehäuseteilen (10, 20) sowie einem in Längsrichtung orientiert dazwischen angeordneten Tragarm (30), welcher an dem einen Ende mit den beiden Gehäuseteilen wirkverbunden und an dem anderen gegenüberliegenden Ende zur Befestigung einer Funktionseinheit ausgebildet ist, wobei die Funktionseinheit (90) einen Stössel (98) sowie damit zusammenwirkende Klemm- oder Schneidelemente (95) umfasst, die beim Zusammendrücken der beiden Gehäuseteile (10, 20) betätigbar sind, wobei eine an dem Tragarm (30) angeordnete und mit den beiden Gehäuseteilen (10, 20) zusammenwirkende Obertragungsvorrichtung (45), mittels welcher die beim Zusammendrücken der beiden Gehäuseteile (10, 20) etwa quer zu dem Tragarm (30) orientierte Bewegung in eine axial in Richtung der Funktionseinheit (90) orientierte Linearbewegung übersetzbar ist und infolge davon ein mit dem einen Ende an der Übertragungsvorrichtung (45) angeordnetes und mit dem anderen Ende mit dem Stössel (98) für die Betätigung der Klemm- oder Schneidelemente (95) zusammenwirkendes Stellglied (40) in axialer Richtung verschiebbar ist, **dadurch gekennzeichnet, dass** die Übertragungsvorrichtung (45) an den einander zugewandten Innenseiten der beiden Gehäuseteile (10, 20) jeweils mit mindestens einer drehbar gelagerten und mit einem Gleitstück (42) des Stellgliedes (40) zusammenwirkenden Laufrolle (27) versehen ist.

2. Chirurgisches Instrument nach Anspruch 1, **dadurch gekennzeichnet, dass** das an dem Schiebebolzen (41) des Stellgliedes (40) angeordnete Gleitstück (42) keilförmig ausgebildet und an der den Laufrollen (27) zugewandten Seite jeweils mit einer geneigten Gleitfläche (43, 43') versehen ist.

3. Chirurgisches Instrument nach Anspruch 2, **dadurch gekennzeichnet, dass** die beiden Gleitflächen (43,43') des keilförmigen Gleitstücks (42) im Profilquerschnitt jeweils als ebene und in Richtung der Spitze geneigte Flächen ausgebildet sind.

4. Chirurgisches Instrument nach Anspruch 2, **dadurch gekennzeichnet, dass** die beiden Gleitflächen (43,43') des keilförmigen Gleitstücks (42) im Profilquerschnitt jeweils als konkave und in Richtung der Spitze geneigte Flächen ausgebildet sind.

5. Chirurgisches Instrument nach Anspruch 2, **dadurch gekennzeichnet, dass** die beiden Gleitflächen (43,43') des keilförmigen Gleitstücks (42) im Profilquerschnitt jeweils als konvexe und in Richtung der Spitze geneigte Flächen ausgebildet sind.

6. Chirurgisches Instrument nach Anspruch 1, **dadurch gekennzeichnet, dass** die beiden Gehäuseteile (10, 20) mit dem hinteren Ende an dem dazwischen angeordneten Tragarm (30) angeordnete und derart befestigt sind, dass diese an dem der Funktionseinheit (90) zugewandten vorderen Ende entgegen einer federelastischen Rückstellkraft relativ zueinander beziehungsweise zu dem Tragarm (30) aufschwenkbar sind.

7. Chirurgisches Instrument nach Anspruch 6, **dadurch gekennzeichnet, dass** die beiden Gehäuseteile (10, 20) an dem hinteren Ende jeweils mit einem plättchenförmig ausgebildeten Federarm (14) sowie einem daran angeformten Endstück (15) an einem Auflageteil (33) des Tragarms (30) angeordnet und befestigt sind.

8. Chirurgisches Instrument nach Anspruch 7, **dadurch** gekennzeichnen, dass der plättchenförmige Federarm (14) als theoretischer Drehpunkt für das einzelne relativ zu dem Tragarm (30 ; 80) aufschwenkbare Gehäuseteil (10, 20) ausgebildet ist.

9. Chirurgisches Instrument nach Anspruch 1, **dadurch gekennzeichnet, dass** das einzelne Gehäuseteil (10, 20) jeweils durch ein am hinteren Ende daran befestigtes und mit einem Absatz (22') in einer zweiten Ausnehmung (32') des zugeordneten Tragarms (30) angeordnetes Sperrglied (23) in Bezug auf den Tragarm (30) gegen seitliches Verschwenken gesichert ist.

10. Chirurgisches Instrument nach Anspruch 1, **dadurch gekennzeichnet, dass** die Funktionseinheit (90,90') eine Führungshülse (91), den koaxial darin angeordneten und mit dem Stellglied (40) zusammenwirkenden Stössel (98), eine Druckfeder (93), eine Stange (92) sowie eine röhrchenförmigen Sonde (96) umfasst, wobei die einzelnen Teile derart miteinander wirkverbunden sind, dass beim Zusammendrücken der beiden Gehäuseteile (10, 20) die röhrchenförmige Sonde (96) relativ zu der feststehenden und am vorderen Ende als Klemmelement (95) ausgebildeten Stange (92) zur Erreichung der Klemmfunktion in axialer Richtung verschiebbar ist.

11. Chirurgisches Instrument nach Anspruch 10, **dadurch gekennzeichnet, dass** beim Zusammendrücken der beiden Gehäuseteile (10, 20) die Stange (92) mit dem daran angeformten Klemmelement (95) in axialer Richtung zur Erreichung der Klemmfunktion in die röhrchenförmige Sonde (96) einziehbar ist.

12. Chirurgisches Instrument nach Anspruch 10, **dadurch gekennzeichnet, dass** das an der Stange (92) angeordnete Klemmelement (95) mit zwei entgegen der federelastischen Rückstellkraft zusammendrückbaren Klemmarmen (94.1, 94.2) versehen ist.

13. Chirurgisches Instrument nach Anspruch 1, **dadurch gekennzeichnet, dass** die Funktionseinheit (90') eine Führungshülse (110), einen darin angeordneten und zur Umwandlung der axialen Schubbewegung in eine Drehbewegung mit einer Kulisse (116.2) wirkverbundenen Wendebolzen (114), eine am vorderen Ende mit einem ersten Schneidblatt (107) versehene Sonde (106) sowie eine am vorderen Ende mit einem zweiten Schneidblatt (108) versehene Stange (109) umfasst, wobei die einzelnen Teile derart zusammenwirken, dass beim Zusammendrücken der beiden Gehäuseteile (10, 20) die Stange (109) mit dem zweiten Schneidblatt (108) um die eigene Längsachse (109') relativ zu dem an der feststehenden Sonde (106) angeordneten ersten Schneidblatt (107) drehbar ist.

14. Chirurgisches Instrument nach Anspruch 1, **dadurch gekennzeichnet, dass** am hinteren Ende eine die beiden Gehäuseteile (10, 20) sowie den dazwischen angeordneten Tragarm (30) umschliessende Verschlusskappe (5) angeordnet und befestigt ist.

15. Chirurgisches Instrument nach Anspruch 14, **dadurch gekennzeichnet, dass** die Verschlusskappe (5) in Abhängigkeit der mit einem Klemmelement (95) oder mit einem Schneidelement (105) versehenen Funktionseinheit (90) eine farbige und visuell zu unterscheidende Oberfläche aufweist.

## Claims

1. A surgical instrument, especially for performing ophthalmological surgeries, consisting of a housing (50) with two oblong housing parts (10, 20) and a support arm (30) which is interposed, oriented in the longitudinal direction and is operatively connected at one end with the two housing parts and is arranged for fastening a functional unit at the other opposite end, with the functional unit (90) comprising a ram (98) and clamping or cutting elements (95) cooperating therewith which can be actuated upon compressing the two housing parts (10, 20), with a transfer apparatus (45) being provided which is arranged on the support arm (30) and cooperates with the two housing parts (10, 20) and by means of which the motion oriented approximately transversally to the support arm (30) during the compression of the two housing parts (10, 20) can be transferred into a linear motion oriented axially in the direction of the functional unit (90) and, as a result of this, an actuating member (40) is displaceable in the axial direction, which member is arranged with the one end on the transfer apparatus (45) and cooperates with the other end with the ram (98) for actuating the clamping or cutting elements (95), **characterized in that** the transfer apparatus (45) is provided on the mutually facing insides of the two housing parts (10, 20) with at least one each of a rotatably held running roller (27) cooperating with a sliding element (42) of the actuating member (40).

2. A surgical instrument according to claim 1, **characterized in that** the sliding element (42) arranged on the sliding pin (41) of the actuating member (40) is provided with a wedge-like arrangement and is provided with an inclined sliding surface (43, 43') each on the side facing the running rollers (27).

3. A surgical instrument according to claim 2, **characterized in that** the two sliding surfaces (43, 43') of the wedge-like sliding element (42) are each arranged in the profile cross section as planar surfaces inclined in the direction towards the tip.

4. A surgical instrument according to claim 2, **characterized in that** the two sliding surfaces (43, 43') of the wedge-like sliding element (42) are each arranged in the profile cross section as concave surfaces inclined in the direction towards the tip.

5. A surgical instrument according to claim 2, **characterized in that** the two sliding surfaces (43, 43') of the wedge-like sliding element (42) are each arranged in the profile cross section as convex surfaces inclined in the direction towards the tip.

6. A surgical instrument according to claim 1, **characterized in that** the two housing parts (10, 20) are arranged with the rear end on the interposed support arm (30) and are fastened in such a way that they are upwardly swivelable relative towards one another or towards the support arm (30) at the front end facing the functional unit (90) against a spring-elastic restoring force.

7. A surgical instrument according to claim 6, **characterized in that** the two housing parts (10, 20) are arranged and fastened to a bearing portion (33) of the support arm (30) at the rear end with a plate-like arranged spring arm (14) and an end piece (15) formed thereon.

8. A surgical instrument according to claim 7, **characterized in that** the plate-like spring arm (14) is arranged as a theoretical center of rotation for the individual housing part (10, 20) which is upwardly swivelable relative to the support arm (30;80).

9. A surgical instrument according to claim 1, **characterized in that** the individual housing part (10, 20) is each secured against lateral swiveling with respect to the support arm (30) by a locking member (23) which is fastened to the rear end of the housing part and is arranged with a step (22') in a second recess (32') of the associated support arm (30).

10. A surgicai instrument according to claim 1, **characterized in that** the functional unit (90, 90') comprises a guide sleeve (91), the ram (98) which is arranged coaxially therein and cooperates with the actuating member (40), a pressure spring (93), a rod (92) and a tubular probe (96), with the individual parts being operatively connected with each other in such a way that upon compressing the two housing parts (10, 20) the tubular probe (96) is displaceable in the axial direction for achieving the clamping function relative to the fixed rod (92) which is arranged at the front end as a clamping element (95).

11. A surgical instrument according to claim 10, **characterized in that** upon compressing the two housing parts (10, 20) the rod (92) with the clamping element (95) formed thereon is retractable in the axial direction into the tubular probe (96) for achieving the clamping function.

12. A surgical instrument according to claim 10, **characterized in that** the clamping element (95) arranged on the rod (92) is provided with clamping arms (94.1, 94.2) compressible against the spring-elastic restoring force.

13. A surgical instrument according to claim 1, **characterized in that** the functional unit (90') comprises a guide sleeve (110), a turning pin (114) which is arranged therein and is operative connected for converting the axial thrusting motion into a rotational motion with a link (116.2) a probe (106) provided at the front end with a first cutting leaf (107) and a rod (109) provided at the front end with a second cutting leaf (108), with the individual parts cooperating in such a way that upon compression of the two housing parts (10, 20) the bar (109) with the second cutting leaf (108) is rotatable about the own longitudinal axis (109') relative to the first cutting leaf (107) arranged on the fixed probe (106).

14. A surgical instrument according to claim 1, **characterized in that** a closing cap (5) is arranged and fastened to the rear end, which cap encloses the two housing parts (10, 20) and the interposed support arm (30).

15. A surgical instrument according to claim 14, **characterized in that** the closing cap (5) has a colored and visually distinguishable surface depending on functional unit (90) provided with a clamping element (95) or a cutting element (105).

## Revendications

1. Instrument chirurgical, en particulier pour la réalisation d'opérations ophtalmologiques, composé d'un boîtier (50) avec deux parties de boîtier allongées (10, 20) et un bras de support (30) orienté dans le sens longitudinal et disposé entre celles-ci, qui est en relation active à une extrémité avec les deux parties de boîtier et qui est conformé à l'extrémité opposée en vue de la fixation d'une unité fonctionnelle, laquelle unité fonctionnelle (90) comprend un poussoir (98) ainsi que des éléments de serrage et de coupe (95) coopérant avec celui-ci, qui peuvent être actionnés lorsque les deux parties de boîtier (10, 20) sont serrées, dans lequel un dispositif de transmission (45) disposé sur le bras de support (30) et coopérant avec les deux parties de boîtier (10, 20), au moyen duquel, lorsque les deux parties de boîtier (10, 20) sont serrées, le mouvement orienté à peu près perpendiculairement au bras de support (30) peut être converti en mouvement linéaire orienté dans le sens axial en direction de l'unité fonctionnelle (90), à la suite de quoi un organe de réglage (40) coopérant à une extrémité avec le dispositif de transmission (45) et à l'autre extrémité avec le poussoir (98) pour actionner les éléments de serrage et de coupe (95) peut être déplacé dans le sens axial, **caractérisé en ce que** le dispositif de transmission (45) est doté sur chacune des faces intérieures tournées l'une vers l'autre des deux parties de boîtier (10, 20) d'au moins un galet de roulement (27) supporté avec possibilité de rotation et coopérant avec une glissière (42) de l'organe de réglage (40).

2. Instrument chirurgical selon la revendication 1, **caractérisé en ce que** la coulisse (42) disposée sur le goujon coulissant (41) de l'organe de réglage (40) est en forme de coin et pourvue sur son côté tourné vers les galets de roulement (27) d'une surface de glissement (43, 43') oblique.

3. Instrument chirurgical selon la revendication 2, **caractérisé en ce que** les deux surfaces de glissement (43, 43') de la coulisse (42) en forme de coin sont conformées en section de profil comme des surfaces planes et obliques en direction de la pointe.

4. Instrument chirurgical selon la revendication 2, **caractérisé en ce que** les deux surfaces de glissement (43, 43') de la coulisse (42) en forme de coin sont conformées en section de profil comme des surfaces concaves et obliques en direction de la pointe.

5. Instrument chirurgical selon la revendication 2, **caractérisé en ce que** les deux surfaces de glissement (43, 43') de la coulisse (42) en forme de coin sont conformées en section de profil comme des surfaces convexes et obliques en direction de la pointe.

6. Instrument chirurgical selon la revendication 1, **caractérisé en ce que** les deux parties de boîtier (10, 20) sont disposées à l'extrémité arrière sur le bras de support (30) disposé entre elles et fixées de telle manière qu'elles puissent pivoter à leur extrémité avant tournée vers l'unité fonctionnelle (90) contre la force de rappel d'un ressort, les unes par rapport aux autres et par rapport au bras de support (30).

7. Instrument chirurgical selon la revendication 6, **caractérisé en ce que** les deux parties de boîtier (10, 20) sont disposées et fixées chacune à l'extrémité arrière sur un bras de ressort (14) en forme de plaquette et une pièce d'extrémité (15) formée sur celui-ci sur un élément d'appui (33) du bras de support (30).

8. Instrument chirurgical selon la revendication 7, **caractérisé en ce que** le bras de ressort (14) en forme de plaquette est conformé comme un centre de rotation théorique pour chaque partie de boîtier (10, 20) pouvant pivoter par rapport au bras de support (30 ; 80).

9. Instrument chirurgical selon la revendication 1, **caractérisé en ce que** chaque partie de boîtier (10, 20) est fixée par un élément de blocage (23) fixé dessus à l'extrémité arrière et disposé par un épaulement (22') dans un deuxième creux (32') du bras de support (30) correspondant par rapport au bras de support (30) afin d'empêcher son basculement latéral.

10. Instrument chirurgical selon la revendication 1, **caractérisé en ce que** l'unité fonctionnelle (90, 90') comprend un manchon de guidage (91), le poussoir (98) disposé dans celui-ci de façon coaxiale et coopérant avec l'organe de réglage (40), un ressort de compression (93), une tige (92) et une sonde tubulaire (96), les différentes parties étant en liaison active les unes avec les autres de telle manière que lorsque les deux parties de boîtier (10, 20) sont serrées, la sonde tubulaire (96) puisse être déplacée dans le sens axial par rapport à la tige (92) fixe et formée à l'extrémité avant pour servir d'élément de serrage (95) afin de réaliser la fonction de serrage.

11. Instrument chirurgical selon la revendication 10, **caractérisé en ce que** lorsque les deux parties de boîtier (10, 20) sont serrées, la tige (92) peut être rétractée dans la sonde tubulaire (96) dans le sens axial avec l'élément de serrage (95) formé dessus pour réaliser la fonction de serrage.

12. Instrument chirurgical selon la revendication 10, **caractérisé en ce que** l'élément de serrage (95) disposé sur la tige (92) est muni de deux bras de serrage (94.1, 94.2) pouvant être serrés ensemble contre la force de rappel élastique d'un ressort.

13. Instrument chirurgical selon la revendication 1, **caractérisé en ce que** l'unité fonctionnelle (90') comprend un manchon de guidage (110), un goujon pivotant (114) disposé dans celui-ci et en liaison active avec une coulisse (116.2) en vue de convertir le mouvement de poussée axiale en mouvement de rotation, une sonde (106) dotée à l'extrémité avant de la première lame de coupe (107) et une tige (109) dotée à l'extrémité avant d'une deuxième lame de coupe (106), ies différentes parties coopérant entre elles de telle manière que lorsque les deux parties de boîtier (10, 20) sont serrées, la tige (109) avec la deuxième lame de coupe (108) puisse tourner autour de son propre axe longitudinal (109') par rapport à la première lame de coupe (107) disposée sur la sonde (106).

14. Instrument chirurgical selon la revendication 1, **caractérisé en ce qu'**un capuchon d'obturation (5) entourant les deux parties de boîtier (10, 20) et le bras de support (30) disposé entre elles est disposé à l'extrémité arrière.

15. Instrument chirurgical selon la revendication 14, **caractérisé en ce que** le capuchon d'obturation (5) présente, selon l'unité fonctionnelle (90) munie d'un élément de serrage (95) ou d'un élément de coupe (105), une surface différente du point de vue de la couleur et de l'aspect.
